# EUROPÄISCHE PATENTANMELDUNG

(11) **EP 0 916 333 A2**
(43) Veröffentlichungstag der Anmeldung: **19.05.1999**
(21) Anmeldenummer: 98121325.9
(22) Anmeldetag: 10.11.1998
(51) Int. Cl.: A61J 3/06

(54) **Tablette und/oder Verpackung für medizinische Anwendungen und/oder für Gesundheitspflege**

(30) Priorität: 14.11.1997 DE 19750416; 24.11.1997 DE 19751808
(71) Anmelder: Cetoni Umweltechnologie-entwicklungsgesellschaft mbH, 94034 Passau (DE)
(72) Erfinder: Schattl, Alice, 94161 Ruderting (DE)
(74) Vertreter: Graf, Helmut, Dipl.-Ing.

(57) **Zusammenfassung**

Die Erfindung bezieht sich auf eine neuartige Tablette und/oder Verpackung für medizinische Anwendungen und/oder für die Gesundheitspflege, mit einer auf ein Organ eine menschlichen oder tierischen Körpers gerichteten Indikation, bestehend aus einem Tablettenkörper.

## Beschreibung

Die Erfindung bezieht sich auf eine Tablette und/oder Verpackung für medizinische Anwendungen und/oder die Gesundheitspflege gemäß Oberbegriff Patentanspruch 1.

Tabletten für medizinische Anwendungen und/oder für die Gesundheitspflege sind in verschiedenen Formen bekannt, und zwar in der Regel in Form von rotationssymetrischen Tabletten-Körpern.

Aufgabe der Erfindung ist es, eine Tablette und/oder Verpackung für medizinische Anwendungen oder für die Gesundheitspflege aufzuzeigen, deren Anwendungsbereich jeweils besonders deutlich erkennbar ist.

Zur Lösung dieser Aufgabe ist eine Tablette und/oder Verpackung für medizinische Anwendungen entsprechend dem Patentanspruch 1 ausgebildet.

Die Besonderheit der erfindungsgemäßen Tablette und/oder Verpackung besteht darin, daß der Tablettenkörper dreidimensional oder die Verpackung entsprechend der Form desjenigen Organs geformt ist, für welches die Tablette oder der Inhalt der Verpackung bestimmt ist, wobei der Begriff 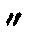 Organe" im Sinne der Erfindung auch Körperteile oder -bereiche, wie z.B. den Kopf eines menschlichen Körpers, aber auch andere Bestandteile, wie Zähne usw. umfaßt.

Die Erfindung hat den Vorteil, daß durch die Form der Tablette und/oder Verpackung die Indikation bzw. Anwendungsbereich deutlich erkennbar ist. Da der gesamte Tablettenkörper dreidimensional bzw. die Verpackung entsprechend der jeweiligen Anwendung geformt ist, ist dieser Anwendungsbereich aus der Form deutlich sichtbar, aber auch fühlbar, und zwar im Gegensatz zu kleinen, in eine neutrale Tablettenform eingeformten Symbolen und Hinweisen, die vielfach nicht oder nur sehr schwer erkennbar sind.

Durch die erfindungsgemäße Ausbildung wird das Einnehmen z.B. der richtigen Tablette für den richtigen Zweck auch für Kinder, ältere oder sehbehinderte Menschen usw. wesentlich vereinfacht.

Die Erfindung eignet sich insbesondere für solche Tabletten, die zur Behebung von allgemeinen Beschwerden z.B. auch rezeptfrei erworben werden können, beispielsweise für Tabletten gegen Kopf- und Zahnweh, gegen Magenverstimmungen, gegen Husten, gegen Halsbeschwerden oder zur Beruhigung. Die Erfindung eignet sich auch für Verpackungen der vorgenannten Tabletten, aber auch für Arzneimittel, Mittel zur Gesundheitspfege usw. in flüssiger Form oder in Pulverform. Weiterbildungen der Erfindung sind Gegenstand der Unteransprüche.

Die Erfindung wird im folgenden näher erläutert, und zwar anhand der Figuren, die als Beispiele verschiedene Tabletten-Formen für unterschiedliche Indikations- oder Anwendungsbereiche wiedergeben.
Fig. 1 zeigt in diesem Sinne eine Tablette 1 in Form eines Herzens. Diese Tablette ist hinsichtlich ihrer medizinischen Wirkstoffe so ausgeführt, daß sie zur Beruhigung eingesetzt wird.
Fig. 2 zeigt eine Tablette 2 in Form eines menschlichen Kopfes. Diese Tablette ist hinsichtlich ihrer medizinischen Wirkstoffe so ausgeführt, daß sie bei Beschwerden oder Schmerzen im Kopfbereich eingesetzt wird.
Fig. 3 zeigt als weiteres Beispiel eine Tablette 3 in Form eines Zahnes. Diese Tablette ist hinsichtlich ihrer medizinischen Wirkstoffe so ausgeführt, daß sie zur Behebung von Beschwerden oder Schmerzen im Zahnbereich dient.
Fig. 4 zeigt eine Tablette 4 in Form eines Magens. Von ihrer Zusammensetzung bzw. von der Verwendung der medizinischen Wirkstoffe hier dient diese Tablette zur Behebung von Magenbeschwerden.

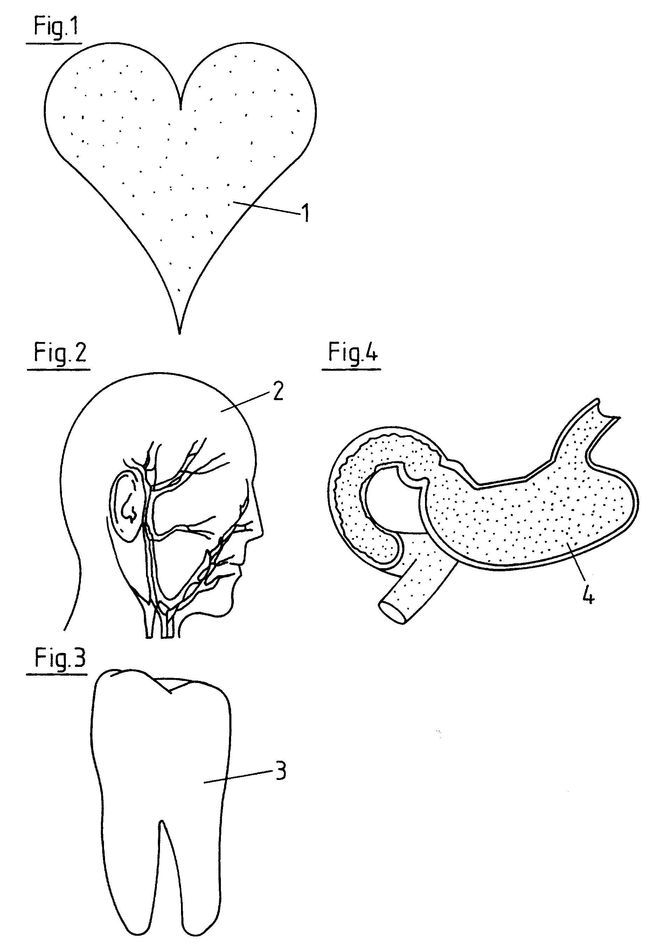

Sämtliche vorgenannten Tabletten 1 - 4 sind bereits durch ihre Form für den jeweiligen Anwendungsbereich deutlich gekennzeichnet. Grundsätzlich besteht auch die Möglichkeit, zwei Formen von Teilen oder Organen des menschlichen oder tierischen Körpers in der Form einer einzigen Tablette zu kombinieren, beispielsweise die Formen der Tabletten 2 und 3, wenn die Indikation sich auf mehrere Teile oder Organe des menschlichen Körpers bezieht.

In gleicher Weise oder in ähnlicher Form, wie dies vorstehend für die Tabletten 1 - 4 beschrieben wurde, können auch Verpackungen für Tabletten oder andere Präparate oder Zubereitungen für medizinische oder gesundheitliche Zwecke ausgebildet sein, wobei die Verpackungen dann beispielsweise insgesamt organbezogen dreidimensional ausgeführt sind. Bei größeren Plister-Verpackungen oder ähnlichen Verpackungen ist es aber auch möglich, beispielsweise nur die Auflageform in ihren Umrissen organbezogen zu formen.

Die Verpackung kann dann aus einem geeigneten Material, beispielsweise auch aus Kunststoff gefertigt sein.

Die Erfindung hat weiterhin auch den Vorteil, daß die Indikation jeweils weltweit sofort erkannt werden kann, insbesondere auch bei im Ausland verkauften Tabletten oder Verpackungen, und zwar ohne daß eine Übersetzung aus einer fremden Sprache notwendig ist.

Verpackungen im Sinne der vorliegenden Erfindung sind zunächst Verpackungen üblicher Art aber auch Verpackungen, die für den Gebrauchszweck notwendig sind, wie z.B. Teebeutel. So ist beispielsweise entsprechend der Erfindung ein Teebeutel für die Behandlung von Margen-Beschwerden ähnlich der Figur 4 entsprechend einem Magen geformt.

Die Erfindung wurde voranstehend an Ausführungsbeispielen beschrieben. Es versteht sich, daß Abwandlungen sowie Änderungen, insbesondere aber auch weitere Formen möglich sind, ohne daß dadurch der der Erfindung zugrundeliegende Erfindungsgedanke verlassen wird.

## Patentansprüche

1. Tablette und/oder Verpackung für medizinische Anwendungen und/oder für die Gesundheitspflege, mit einer auf ein Organ eines menschlichen oder tierischen Körpers gerichteten Indikation, bestehend aus einem Tablettenkörper, **dadurch gekennzeichnet**, daß der Tablettenkörper insgesamt in einer der Form des jeweiligen Organs entsprechenden Form geformt ist, auf welches sich die Indikation der Tablette bezieht, und/oder daß die Verpackung in einer der Form des jeweiligen Organs entsprechenden Form geformt ist, auf welches sich die Indikation des Inhalts der Verpackung bezieht.

2. Tablette und/oder Verpackung nach Anspruch 1, dadurch gekennzeichnet, daß der Tablettenkörper und/oder die Verpackung eine Herzform aufweisen.

3. Tablette und/oder Verpackung nach Anspruch 1, dadurch gekennzeichnet, daß der Tablettenkörper und/oder die Verpackung entsprechend einem menschlichen Kopf geformt ist.

4. Tablette und/oder Verpackung nach Anspruch 1, dadurch gekennzeichnet, daß der Tablettenkörper und/oder die Verpackung entsprechend einem menschlichen Zahn geformt ist.

5. Tablette und/oder Verpackung nach Anspruch 1, dadurch gekennzeichnet, daß der Tablettenkörper und/oder die Verpackung entsprechend einem Magen geformt ist.
